# EUROPEAN PATENT APPLICATION

(11) **EP 2 030 636 A2**
(43) Date of publication of application: **04.03.2009**
(21) Application number: 08251153.6
(22) Date of filing: 28.03.2008
(51) Int. Cl.: A61L 2/00, A61L 2/08, A61L 2/14

(54) **Self-sterilizing device**

(30) Priority: 17.08.2007 US 894031
(71) Applicant: Searete LLC, Bellevue, WA 98004 (US)
(72) Inventor: Dacey, Ralph G. Jnr., St. Louis, MO 63124 (US); Hyde, Roderick A., Redmond, WA 98052 (US); Ishikawa, Muriel Y., Livermore, CA 94550-4921 (US); Leuthard, Eric C., St. Louis, MO 63130 (US); Myhrvold, Nathan P., Bellevue, WA 98004 (US); Rivet, Dennis J. II, Portsmouth, VA 23704 (US); Smith, Michael A., Phoenix, AZ 85050 (US); Tegreene, Clarence T., Bellevue, WA 98004-2834 (US); Wood, Lowell L. Jr., Bellevue, WA 98004 (US); Wood, Victoria Y. H., Livermore, CA, 94550-4921 (US)
(74) Representative: Harris, Ian Richard

(57) **Abstract**

An insertable medical element may be supportive of evanescent energy for sterilization of a biomaterial. A system including an insertable medical element supportive of evanescent energy may include an energy source, where the energy source may be emissive of electromagnetic or plasmon energy, a sensor such as a surface plasmon resonance sensor, and/or it may include an imager.

## Description

### SUMMARY

In one embodiment, a method of establishing a sterile region in an insertable medical element comprises generating at at least one surface region of the insertable medical element an evanescent field having properties selected to substantially disable biomaterial in the at least one surface region.

In another embodiment, a method comprises guiding electromagnetic energy along a fluid passageway at least partially within a patient, generating plasmon energy along the fluid passageway responsive to the guided electromagnetic energy, and delivering a biomaterial through the fluid passageway after or during the generating plasmon energy along the fluid passageway responsive to the guided electromagnetic energy.

In another embodiment, an apparatus comprises a first biofluid guiding conduit extending along a path from a first location to a second location, wherein at least a portion of the first biofluid guiding conduit is insertable into a patient, a guiding structure configured to guide electromagnetic energy proximate to at least a portion of the path, and a conversion structure operative to convert electromagnetic energy to plasmon energy, the conversion structure being positioned to receive the guided electromagnetic energy and to provide the plasmon energy to the portion of the path.

In another embodiment, a system comprises a light source having an output energy, an insertable medical element supportive of evanescent energy, and an evanescent field generator coupled to receive the output energy and responsive to produce the evanescent energy within or proximate a biomaterial at a sterilization level.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE FIGURES

Embodiments are described hereinafter with reference to the accompanying drawings.
**Figure 1** is a schematic of a plasmon at a boundary.
**Figure 2** is a schematic of an embodiment of an apparatus supportive of plasmon energy.
**Figure 3** is a schematic of an embodiment of an apparatus having first and second fluid guiding conduits.
**Figure 4** is a schematic of an embodiment of an apparatus supportive of plasmon energy.
**Figure 5** is a schematic of an embodiment of a system including an insertable medical element.
**Figure 6** is a schematic of a fluid guide.
**Figure 7** is a schematic of an embodiment of a system including an imaging system.
**Figure 8** is a flow chart depicting a method.

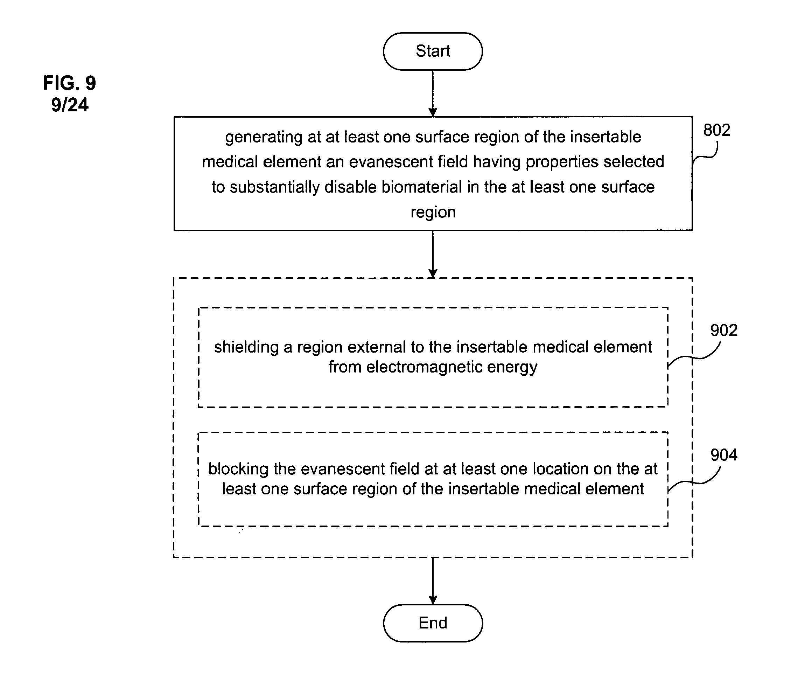

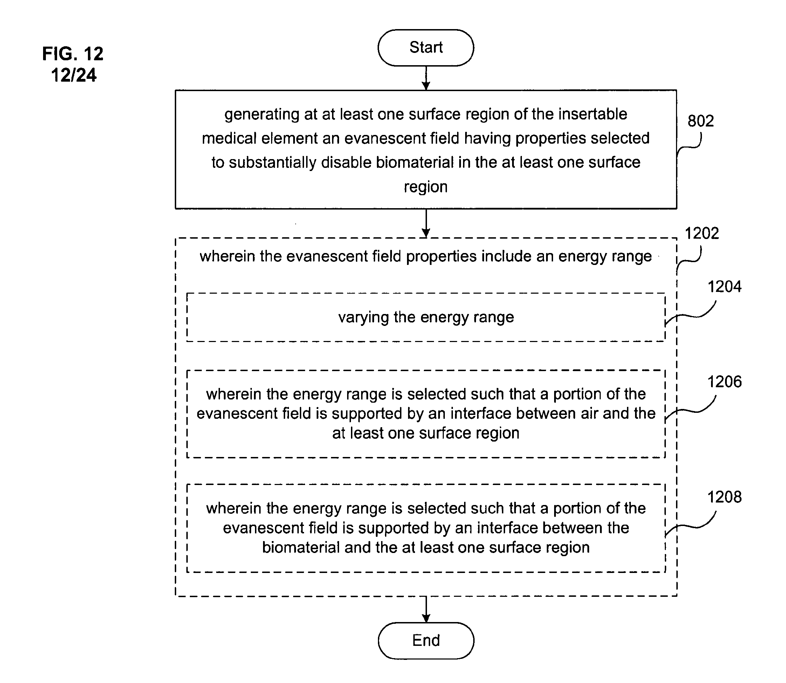

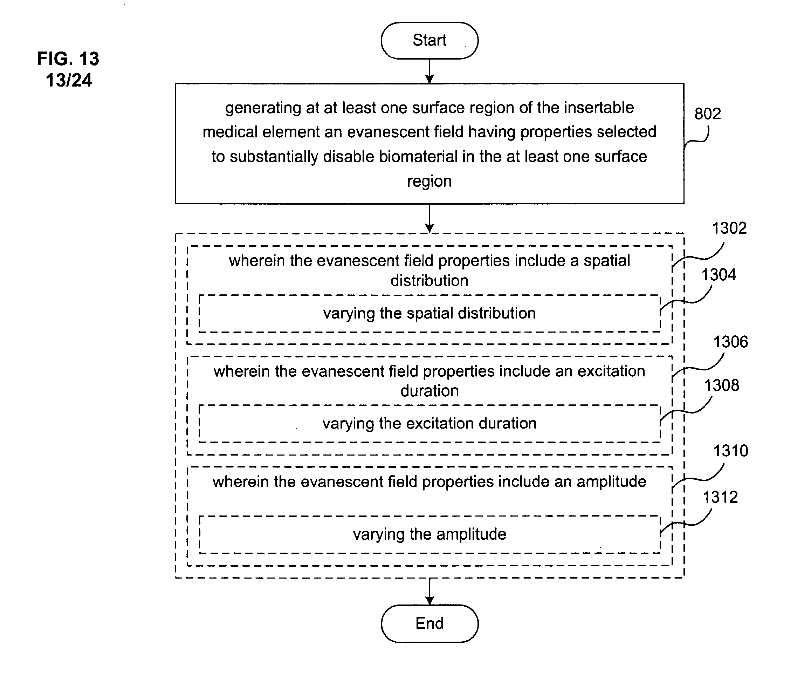

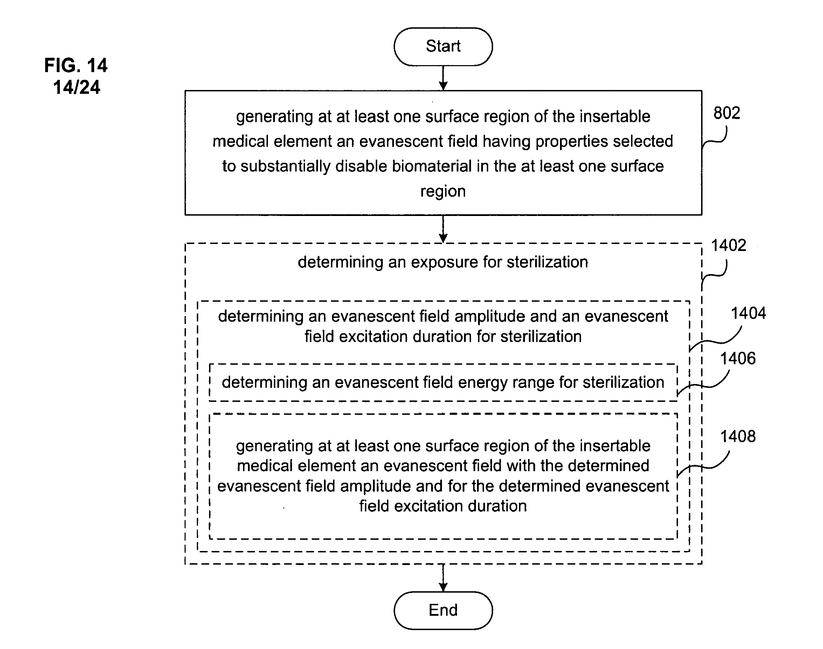

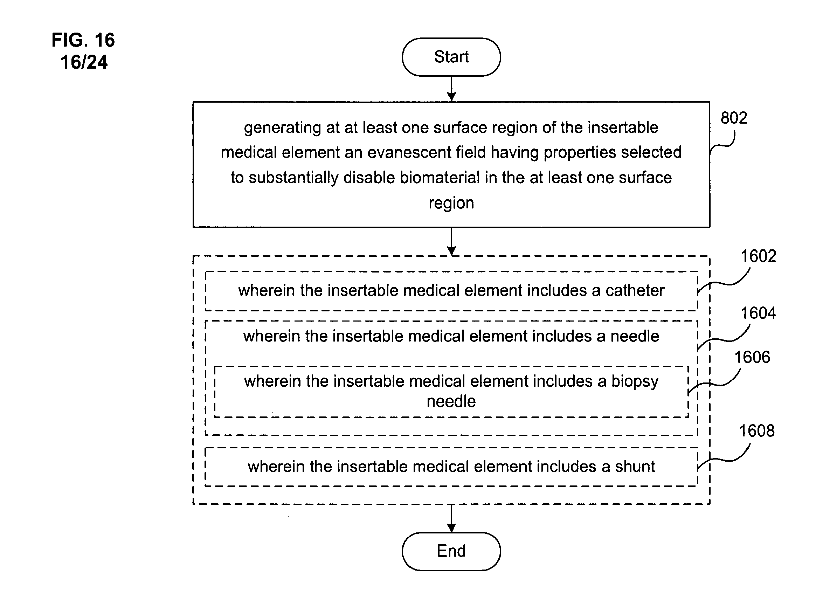

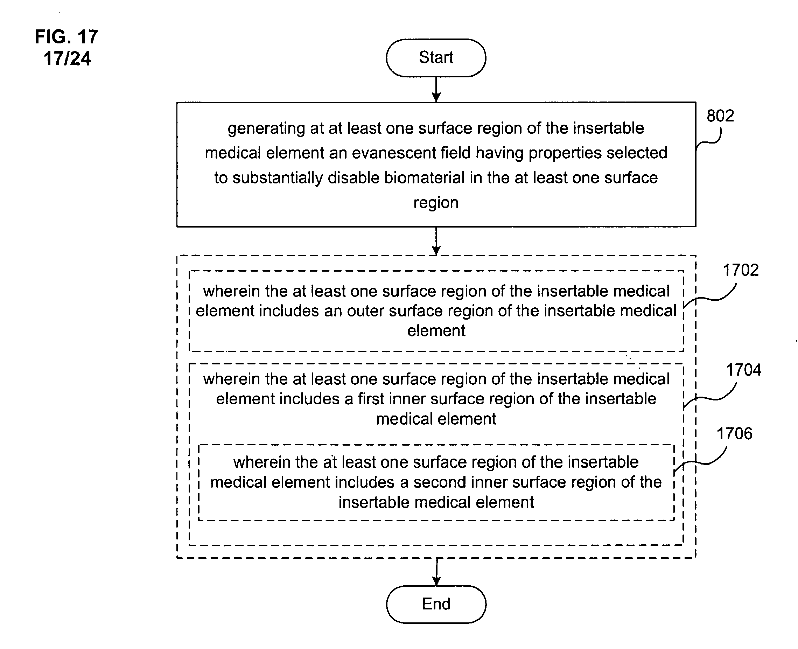

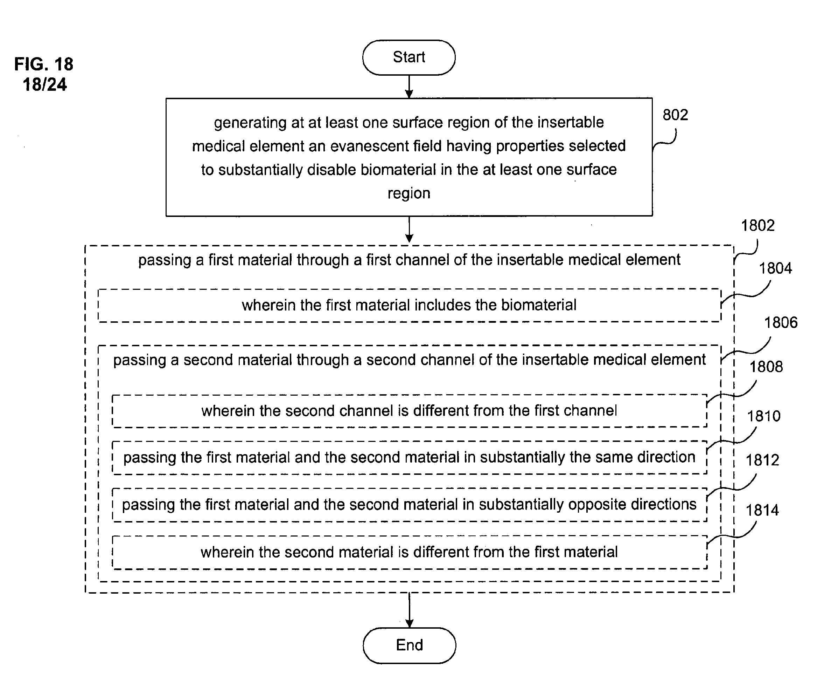

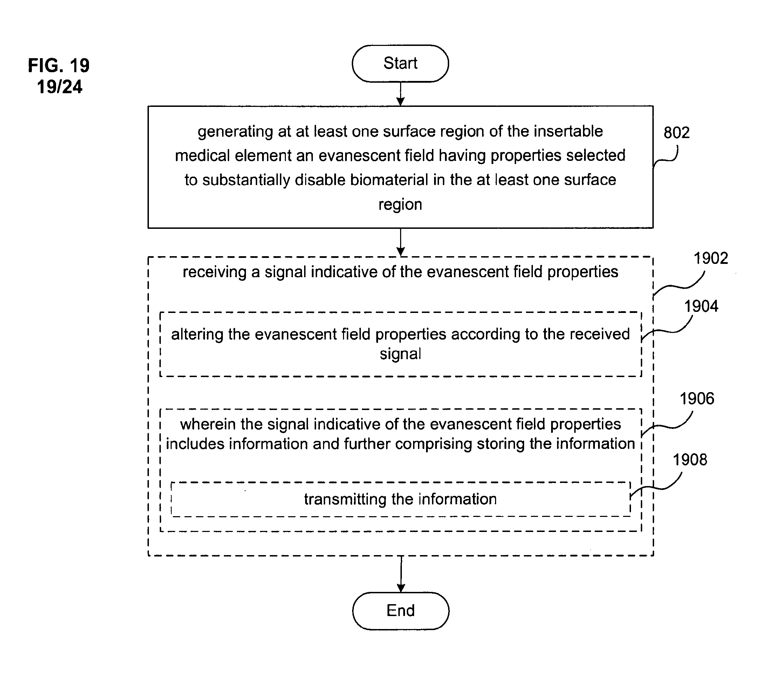

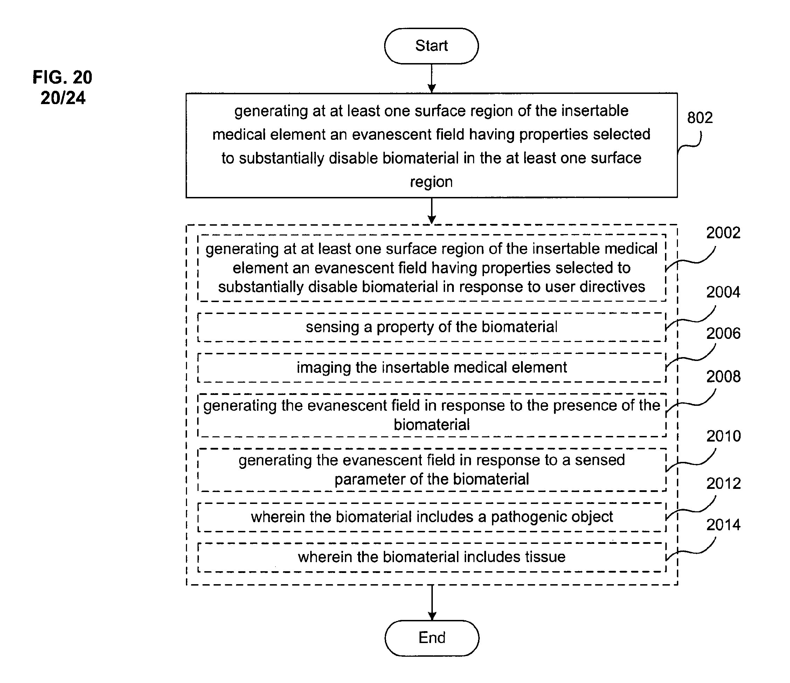

**Figures 9-20** depict variants of the flow chart of Figure 8.

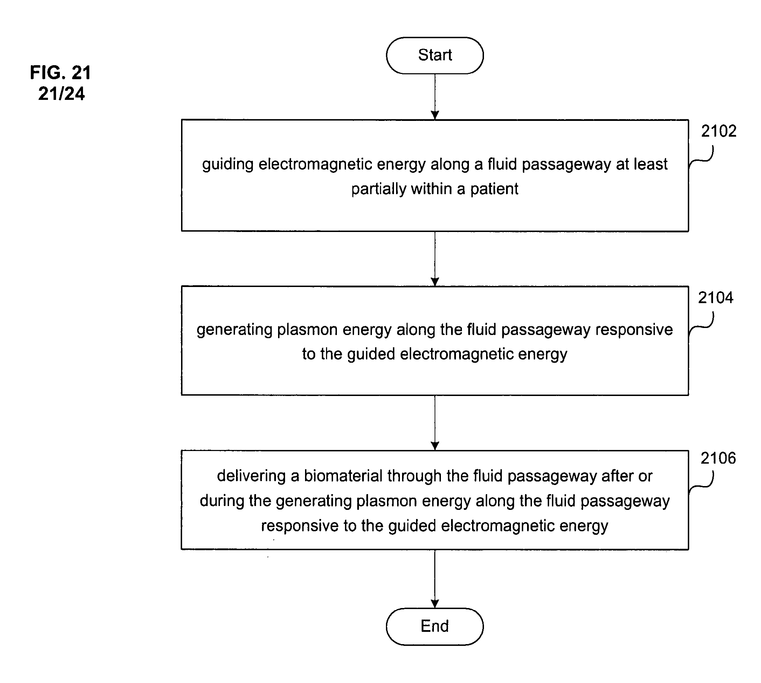

**Figure 21** is a flow chart depicting a method.

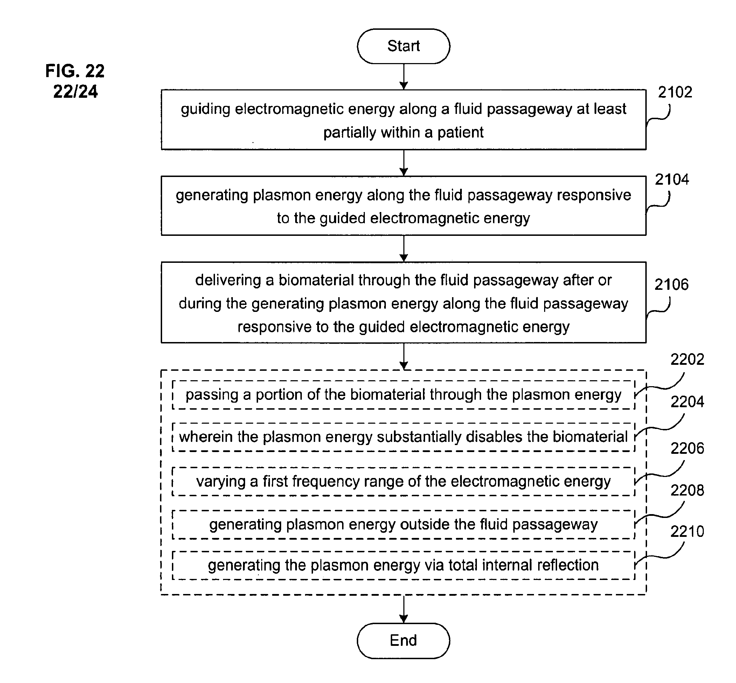

**Figures 22-24** depict variants of the flow chart of Figure 21.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented here.

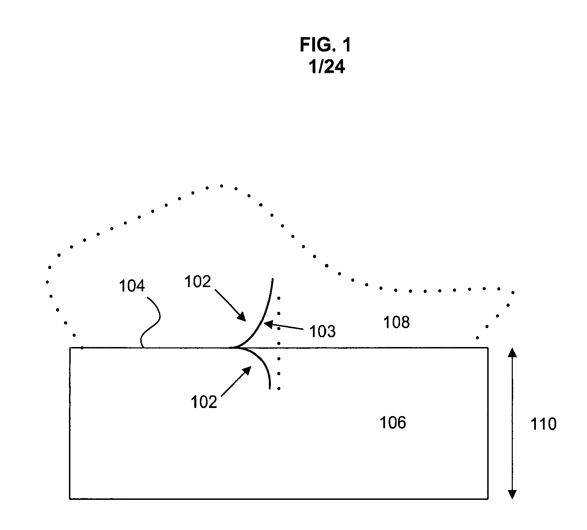

Surface plasmons may exist on a boundary between two materials when the real parts of their dielectric constants ε and ε' have different signs, for example between a metal and a dielectric. Figure 1 shows a plasmon 102 at a boundary 104 of a material 106 having a negative real dielectric constant, such as a metal. The material or structure 108 forming the boundary 104 with the material 106 may be: air, vacuum, or its equivalent; a substantially homogeneous dielectric material; or a different material or structure. The boundary 104, although shown as being substantially continuous and planar, may have a different shape. The plasmon 102, although shown as including substantially exponential functions with a field maximum at the boundary 104, may include only approximately exponential functions, may be described by a different function, and/or may have a field maximum someplace other than the boundary. Further, although the plasmon 102 is shown at a certain location on the boundary 104 for illustrative purposes, the spatial distribution of the plasmon 102 may be anything.

In some embodiments the material thickness 110 may be smaller than the plasmon wavelength, as described in Alexandra Boltasseva, Thomas Nikolajsen, Krisjan Leosson, Kasper Kjaer, Morten S. Larsen, and Sergey I. Bozhevolnyi, "INTEGRATED OPTICAL COMPONENTS UTILIZING LONG-RANGE SURFACE PLASMON POLARITONS", Journal of Lightwave Technology, January, 2005, Volume 23, Number 1, which is incorporated herein by reference. Further, Boltasseva describes how a metal may be embedded in a dielectric to allow propagation of long-range surface plasmon polaritons, where the parameters of the metal, including thickness 110 and width (not shown), may control the propagation of the plasmon.

The plasmon 102 includes an evanescent field 103, where the evanescent field 103 is the portion of the plasmon 102 extending into the material or structure 108. However, an evanescent field may occur outside of a surface plasmon. For example, an evanescent field may occur at the boundary between two dielectrics where total internal reflection occurs. The evanescent field may provide energy to a biomaterial, for example, for sterilization, as described in the following embodiments.

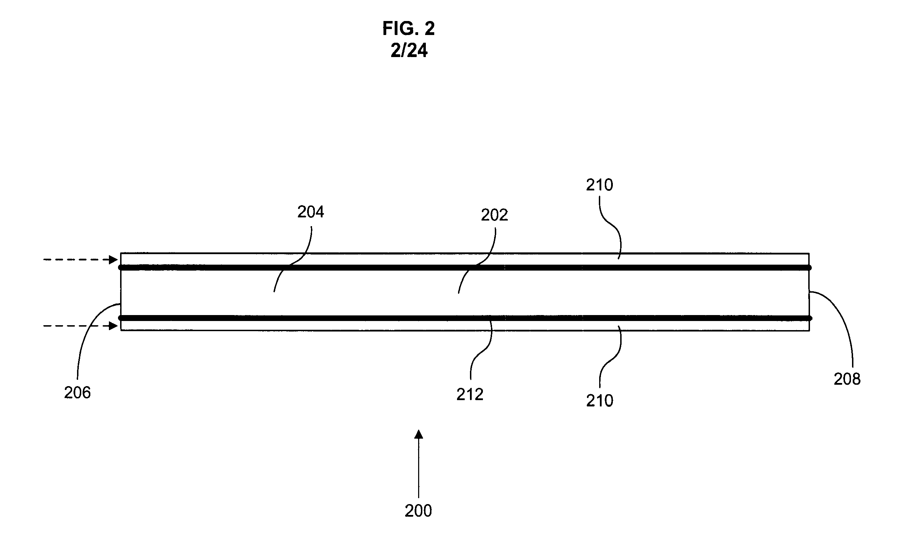

Figure 2 shows a side-cross-sectional view of an embodiment of an apparatus 200 supportive of plasmon energy. The embodiment 200 comprises a first biofluid guiding conduit 202 (such as a hollow tube) extending along a path 204 from a first location 206 to a second location 208, wherein at least a portion of the first biofluid guiding conduit 202 is insertable into a patient; a guiding structure 210 (for example, an electromagnetically transmissive dielectric) configured to guide electromagnetic energy proximate to at least a portion of the path 204; and a conversion structure 212 (for example, a metal coating) operative to convert electromagnetic energy to plasmon energy, the conversion structure 212 being positioned to receive the guided electromagnetic energy and to provide the plasmon energy to the portion of the path 204.

In the embodiment shown, the guiding structure 210 is configured to allow transmission of electromagnetic energy. The electromagnetic energy can create plasmons on the conversion structure 212 via total internal reflection, and the plasmon field may extend into the first biofluid guiding conduit 202. This plasmon field may then be used to sterilize fluid in the first biofluid guiding conduit 202.

The guiding structure 210 may include, for example, an optically transmissive material such as glass, plastic, or a different type of material or structure. Although the guiding structure 210 is shown in Figure 2 as having a single layer, in some cases the guiding structure 210 may include multiple layers. For example, the guiding structure 210 may include layers of glass and plastic, or layers of other materials having different indices of refraction. These materials may be chosen based on the guiding properties of the assembly.

The guiding structure 210 may substantially surround the first biofluid guiding conduit 202, may be substantially parallel to the first biofluid guiding conduit 202, or may have a different configuration relative to the first biofluid guiding conduit 202. For example, as shown in Figure 2, the guiding structure 210 substantially surrounds the first biofluid guiding conduit 202. However, in other embodiments the guiding structure 210 may be a strip of material proximate to the first biofluid guiding conduit 202, or may have another orientation relative to the first biofluid guiding conduit 202.

The conversion structure 212 may include a conductive coating such as silver or a different conductor. For example, as shown in Figure 2, the conversion structure 212 is a conductive coating that substantially surrounds the first biofluid guiding conduit 202 and separates the first biofluid guiding conduit 202 from the guiding structure 210. However, in other embodiments the conversion structure 212 may have a different configuration. For example, where the guiding structure 210 is a strip of material proximate to the first biofluid guiding conduit 202, the conversion structure 212 may be a conductive strip between the guiding structure 210 and the first biofluid guiding conduit 202. Further, the conversion structure 212 may be an aggregate of particles supportive of plasmons, a grating, or another device configured to convert electromagnetic energy to plasmons, and need not be a continuous conductive strip as shown in the example embodiment of Figure 2. The conversion structure 212 may convert electromagnetic energy to an evanescent field, where in this case the conversion structure may be a dielectric arranged to produce evanescent energy via total internal reflection, a diffraction grating having a period smaller than the wavelength of the electromagnetic energy, or it may have a different configuration. The conversion structure 212 and the first biofluid guiding conduit 202 may in some cases be the same device, such as the case where conductive material forms the boundary of the first biofluid guiding conduit 202. Or, the conversion structure 212 may cover just a portion of the inner wall surface of the first biofluid guiding conduit.

The embodiment 200 of the apparatus may be part of a larger apparatus, for example, a needle, a catheter, or another device that is at least partially insertable into a patient.

Although the embodiment 200 of the apparatus shown in Figure 2 is configured such that evanescent energy extends within the first biofluid guiding conduit 202, in other embodiments the apparatus 200 may be configured so that evanescent energy extends externally to the apparatus 200, for example, where the conversion structure 212 substantially surrounds the guiding structure 210. Or, the apparatus 200 may be configured such that evanescent energy extends both outside the apparatus 200 and inside the first biofluid guiding conduit 202. Where evanescent energy extents outside the apparatus 200, the evanescent energy may disable a biomaterial (for example, bacteria and viruses) on the surface of the apparatus 200, and may be used for sterilizing the apparatus 200. This may be done prior to insertion of the apparatus 200 into a patient.

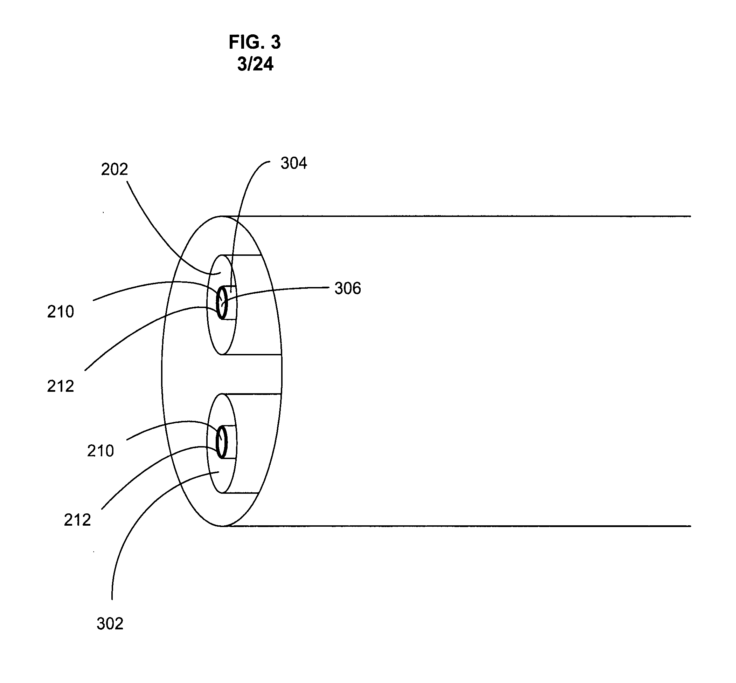

In one embodiment, a cross section of which is shown in Figure 3, the first biofluid guiding conduit 202 includes a wall 304 defining a passageway 306, where the passageway 306 is configured to support electromagnetic energy transmission. For example, in Figure 3, the wall 304 forming the passageway 306 is the wall of an optical fiber, where the optical fiber forms the guiding structure 210 configured to support electromagnetic energy transmission. In this case, the conversion structure 212 includes a conductive coating on the guiding structure 210. Although the embodiment is shown and described such that the first biofluid guiding conduit 202 has a single passageway 306 supportive of electromagnetic energy, in other embodiments the first biofluid guiding conduit 202 may have more than one passageway 306 supportive of electromagnetic energy, where each passageway 306 may include a conversion structure 212 arranged to convert electromagnetic energy to plasmon energy.

The embodiment in Figure 3 further includes a second biofluid guiding conduit 302. Each of the first and second biofluid guiding conduits 202, 302 may be configured according to the description of the first biofluid guiding conduit 202 shown in Figure 2 with a guiding structure 210 and a conversion structure 212. The first and second biofluid guiding conduits 202, 302 may be configured to carry different fluids, may be configured to support plasmons having different energies, and/or may have other differences. For example, first biofluid guiding conduit 202 may support fluid flowing in one direction and the second biofluid guiding conduit 302 may support fluid flowing in an opposite direction.

Although the embodiment is shown having two biofluid guiding conduits 202, 302, in other embodiments there may be a different number of biofluid guiding conduits. Further, although the biofluid guiding conduits 202, 302 are shown as being substantially parallel and the same size, in other embodiments they may not be parallel and/or they may have different sizes. The first and second biofluid guiding conduits 202, 302 may each include a separate guiding structure 210 and/or conversion structure 212 as shown in Figure 3 and may share a common optical source or may be driven independently. Such an embodiment may permit selective sterilization of individual conduits such as 202, 302. In one approach, the conduits 202, 302 are isolated by an intermediate layer that forms a barrier to allow independent generation of plasmons for each of the channels.

In a different embodiment the first and second biofluid guiding conduits 202, 203 may be configured to share a guiding structure 210 and/or conversion structure 212.

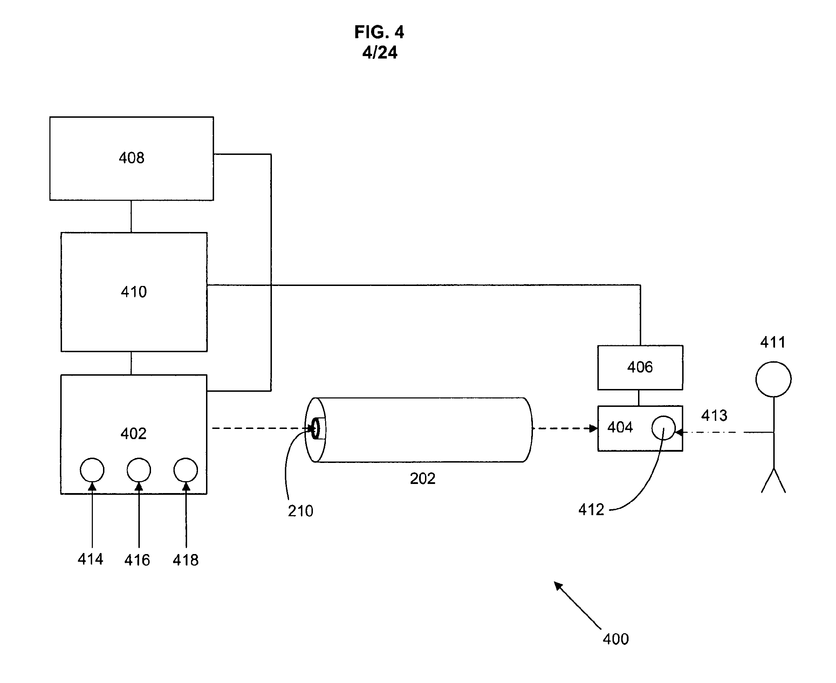

Figure 4 shows an embodiment of an apparatus 400 comprising a generator 402 arranged to produce electromagnetic energy, where in this case the electromagnetic energy is incident on the guiding structure 210, and where the guiding structure is inside the first biofluid guiding conduit 202. The generator 402 may include selectors 414, 416, 418, where in this embodiment the selectors include an amplitude range selector 414, a duration selector 416, and an energy range selector 418.

For example, where the generator 402 is configured to output electromagnetic energy in the ultraviolet portion of the electromagnetic spectrum, the energy range selector 418 may be configured to select energies substantially in this range. Or, the generator 402 may be configured to output electromagnetic energy in the visible portion of the electromagnetic spectrum, and the energy range selector 418 may be configured to select energies substantially in this range. Although energies in the ultraviolet and visible ranges are described here as example embodiments, in other embodiments the generator 402 may be configured to output electromagnetic energy in a different portion of the spectrum, or in several different portions of the spectrum (for example, both visible and ultraviolet energy).

The duration selector 416 may be configured to select a time range for which the generator 402 is on, and/or a time pattern for an on/off cycle of the generator 402 to follow, and/or some other selection of time distribution for the operation of the generator 402. For example, the selector 416 may allow a user to set the generator 402 to be on for two minutes every hour, or for several hours a day, or following some other pattern. Or, a user may set the generator to be on for ten seconds following the flow of fluid through the first biofluid guiding conduit 202, for example. Many different temporal distributions may be desired and one skilled in the art may adjust the selector 416 to accommodate these distributions.

Although three different selectors 414, 416, 418 are shown, other embodiments may include more, less, or different selectors. In some embodiments the selectors 414, 416, 418 may be knobs allowing for user selection as shown in Figure 4, or they may be configured to receive an electronic or other signal, or they may be controlled in a different way.

The apparatus 400 shown in Figure 4 may further comprise a receiver 404 positioned to receive the energy, where the receiver 404 may be configured to receive electromagnetic and/or plasmon energy. The apparatus 400 may further comprise a transmitter 406 arranged to transmit information related to the received energy. The apparatus 400 may further comprise a storage medium 408 arranged to store information related to the received energy, a processor 410 arranged to process information related to the received energy. Although the generator 402, receiver 404, transmitter 406, storage medium 408, and the processor 410 are shown in Figure 4 as separate units, in some embodiments some or all of them may be incorporated into a single device. For example, in some embodiments the receiver 404 and the transmitter 406 may be incorporated into a single unit, the storage medium 408 and the processor 410 may be incorporated into a single unit, or there may be other configurations and one skilled in the art will recognize that there are many permutations of the configuration shown in Figure 4.

The embodiment shown in Figure 4 shows electromagnetic energy produced by a generator 402, traveling through the first biofluid guiding conduit 202, and then being received by the receiver 404. However in other embodiments the first biofluid guiding conduit 202 may include one or more reflectors such that electromagnetic energy remains substantially trapped in the first biofluid guiding conduit 202. Or, the electromagnetic energy may leave the first biofluid guiding conduit 202 in a highly dispersed fashion and not in the collimated beam as shown in Figure 4. The actual path taken by electromagnetic energy incident on the first biofluid guiding conduit 202 is a function of, for example, the structure, optical elements included, and other properties of the first biofluid guiding conduit 202, and one skilled in the art may find many variants on Figure 4.

Figure 4 further shows an input port 412 arranged to receive a first signal 413 directive of information related to the received energy. The input port 412 is shown in Figure 4 as a knob where the first signal 413 is a selection made by a user 411 (not drawn to scale), where a user 411 may turn the knob to select, for example, an energy range (for example, a subset of energies in the ultraviolet portion of the spectrum such as 8-9 eV, or a subset of energies in the visible portion of the spectrum such as 2-3 eV, or a different energy range) to receive. Although the input port 412 is described as receptive to an energy range selection, in other embodiments the input port 412 may be receptive to a different selection, such as a time duration for receiving energy or another type of selection. Or, there may be more than one input port 412, where the different input ports may be receptive to different signals. Although the input port 412 is described as a knob and the first signal 413 is described as a user selection, in other embodiments the input port 412 and first signal 413 may be a different combination, such as the case where the input port 412 is an electronic port and the input signal is an electronic signal that may be configured to, for example, sweep through a range of frequencies to receive. The input port 412 may have yet a different configuration and the input signal may take a different form, and one skilled in the art may recognize a variety of different ways of inputting information into the input port 412.

Figure 4 includes a multitude of different devices such as the generator 402, receiver 404, transmitter 406, storage medium 408, and processor 410. However in some embodiments only one or a few of the devices 402, 404, 406, 408, 410 may be included. For example, one embodiment may include a generator 402 but not other devices such as the receiver 404, or there may be a different combination of devices included. Further, some embodiments may include other devices or components not shown.

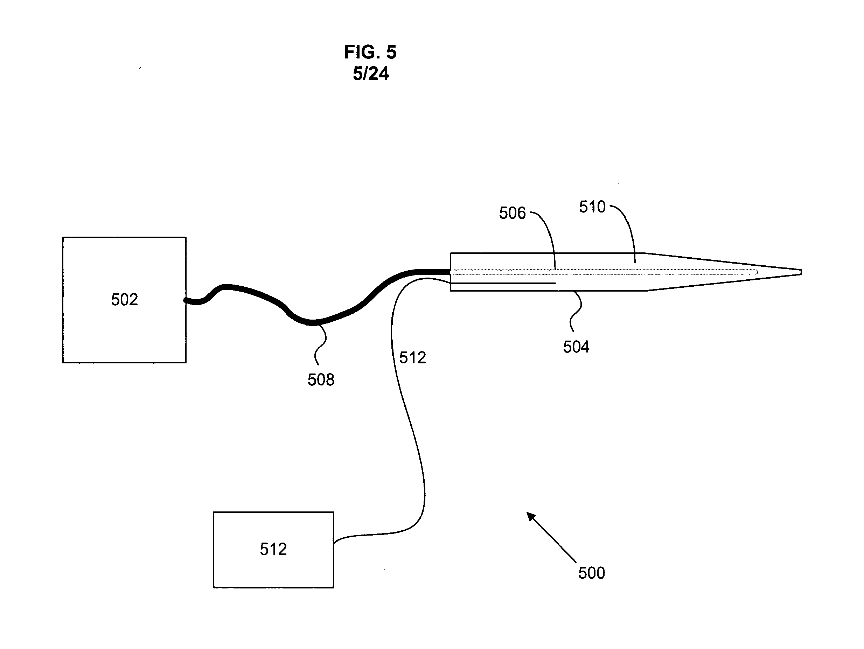

In an embodiment shown in Figure 5, a system 500 comprises a light source 502 having an output energy, an insertable medical element 504 supportive of evanescent energy, and an evanescent field generator 506 coupled to receive the output energy and responsive to produce the evanescent energy within or proximate a biomaterial at a sterilization level. The system 500 further comprises a sensor 512, where the sensor 512 may be a surface plasmon resonance sensor.

In this embodiment the insertable medical element 504 is a needle configured to deliver a biomaterial such as a vaccine to a patient, where the insertable medical element 504 includes a fluid guide 510 configured to carry the biomaterial. The evanescent field generator 506 is the outer surface of an optical fiber 508 that is configured to produce an evanescent field. Although the insertable medical element 504 is shown and described as a needle configured to deliver a biomaterial, in other embodiments the insertable medical element may include a catheter, a biopsy needle, an implantable device (including a cardiac modulation device, a neuromodulation device such as a spinal cord stimulator or intrathecal pain pumps, and/or a different implantable device), a shunt, an electrode, an external bone fixation device, or a different type of element. The device(s) may be configured for temporary or substantially permanent insertion into the patient, and/or may be configured to be partially or fully insertable into the patient. Further, although the insertable medical element 504 is described as a needle configured to deliver a biomaterial to a patient, in some embodiments the insertable medical element 504 may be a needle configured to receive or extract a biomaterial.

Although this embodiment is described as being configured to produce an evanescent field in the insertable medical element 504 via total internal reflection, other embodiments may be configured to produce an evanescent field via a plasmon. For example, where the portion of the fiber 508 that is inside the insertable medical element 504 is coated with a conductor, plasmons may be generated on the conductor. Or, in another embodiment, evanescent fields may be configured to occur both by total internal reflection and via a plasmon.

Although the sensor 512 is shown as being independent from the fiber 508, in other embodiments the sensor 512 and the fiber 508 may be integrated together.

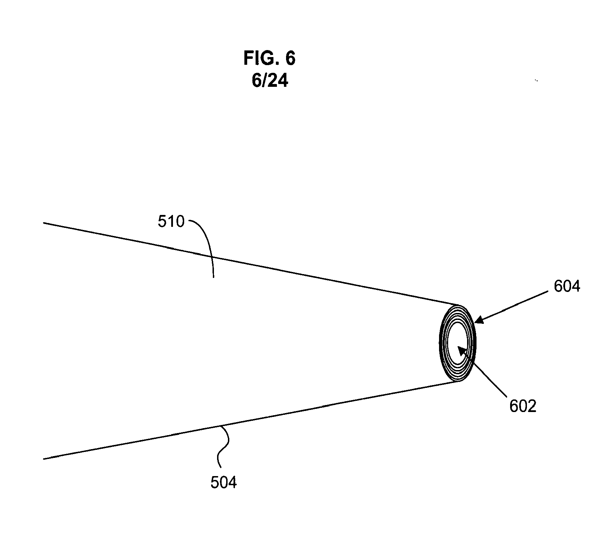

The fluid guide 510 may further comprise an aperture 602, shown in detail in Figure 6, where the aperture 602 may include a grating 604 proximate to the aperture 602 and where the grating 604 is configured to produce a plasmon field proximate to the aperture, such that when the biofluid flows through the aperture, the biofluid is sterilized by the plasmon field.

Although the aperture 602 is depicted in Figure 6 as being round, in other embodiments it may have another shape. For example, the aperture 602 may be a substantially rectilinear shape such as a slit. Further, the grating 604 is depicted as also being round, but it too may have a different shape, such as in the case where the aperture 602 is a slit and the grating 604 may include a series of lines surrounding the slit. Some embodiments may further include a multitude of apertures 602 to facilitate greater fluid flow.

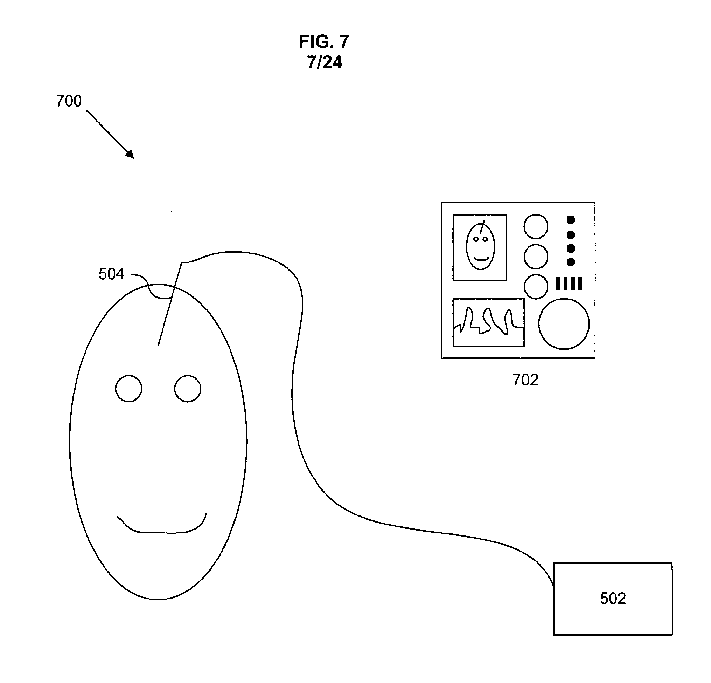

In another embodiment a system 700 may comprise an imaging system 702 as shown in Figure 7, wherein the imaging system 702 may be operable to image the insertable medical element 504 as shown. Figure 7 shows an imaging system 702 such as an x-ray imager that is not coupled by wires or other connectors to the insertable medical element 504. However in some embodiments the imaging system 702 may be connected to the insertable medical element 504 via fiber optic cables, wires, or a different connector.

The imaging system may include an optical imaging system, an ultrasound system, an MRI system, a radiography system, or a different kind of imaging system. The imaging system may be operative to detect biomaterial. Further, the insertable medical element 504 may include a portion of the imaging device, for example, where the imaging system 702 includes fiber optic cables configured to image the area of the body around the insertable medical element 504. The imaging system 702 may be configured to image the insertable medical element 504, for example, to position the element. Or, the imaging system 702 may be configured to image tissue surrounding the medical element to determine the character of the tissue or for another reason.

The embodiments as previously described may further include a biocompatible material. For example, with reference to Figure 5, the insertable medical element 504 may include a coating of a biocompatible material.

Although the embodiments described above generally show configurations supporting plasmons and/or evanescent fields inside of a device (as, for example, in Figure 3, where the conversion structure 212 is inside the first biofluid guiding conduit 202), other embodiments may be configured to support plasmons and/or evanescent fields on the outside of a device. For example, with reference to Figure 5, the insertable medical element 504 may have a conversion structure 212 configured to produce plasmons to sterilize biomaterial on the outside of the device. This may be useful in situations where, for example, the insertable medical element 504 enters the body and infection may be likely to occur.

Further, the embodiments as previously described may include an electromagnetic shield. For example, again with reference to Figure 5, the insertable medical element may include a coating of a shielding material configured to shield the patient from electromagnetic energy.

For clarity of presentation the embodiments described above may show portions of apparatuses and may include other components not shown. For example, the embodiments shown generally do not show apparatus configured to guide fluid to or from the first biofluid guiding conduit 202 and may not show apparatus such as optics and/or waveguides configured to guide the electromagnetic energy to or from the guiding structure 210, however some embodiments may include such apparatus.

The previously described embodiments are generally configured to support evanescent energy on a surface that is substantially perpendicular to the direction of a fluid flow, for example, as in Figure 5 where the evanescent field generator 506 includes a fiber that extends axially along a central portion of the insertable medical element 504. However there are many other configurations that support evanescent energy and it is not necessary for the evanescent field generator to have cylindrical or other symmetry and there are many different orientations the evanescent field generator 506 may have relative to the insertable medical element 504.

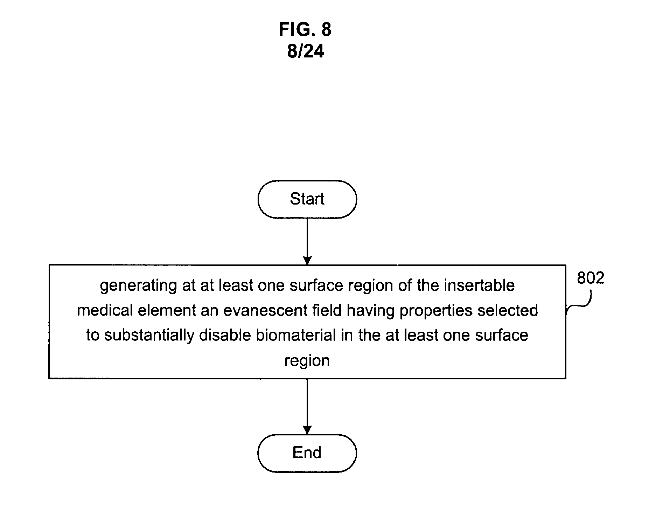

In one embodiment, a method of establishing a sterile region in an insertable medical element, shown in the flow chart of Figure 8, comprises (802) generating at at least one surface region of an insertable medical element an evanescent field having properties selected to substantially disable biomaterial in at least one surface region.

The method may further comprise (902) shielding a region external to the insertable medical element from electromagnetic energy, which may include blocking the electromagnetic energy with a reflector, an absorber, or a different type of device. The method may further comprise (904) blocking the evanescent field at at least one location on the at least one surface region of the insertable medical element, which may include blocking the evanescent field with a reflector, an absorber, or another type of device.

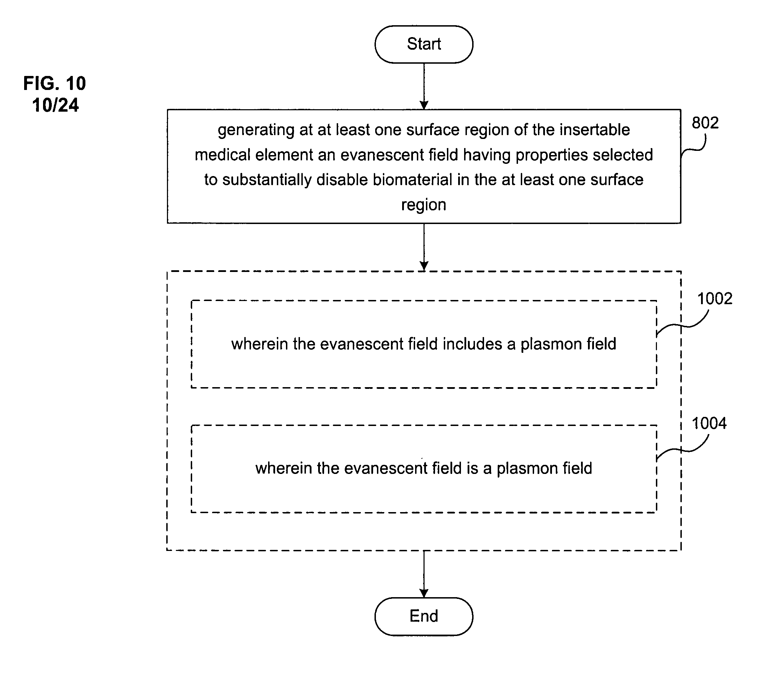

In one case, (1002) the evanescent field may include a plasmon field, and in another case, (1004) the evanescent field may be a plasmon field, as shown in Figure 10.

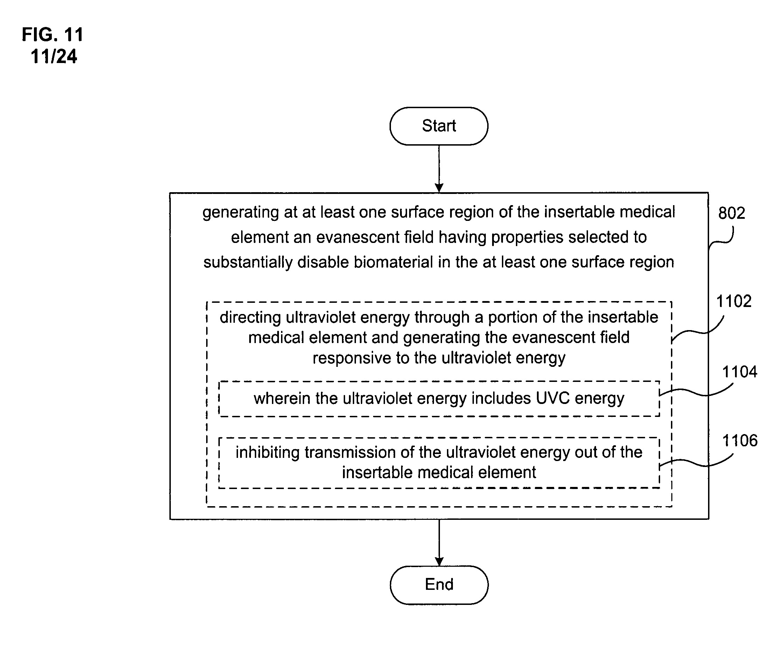

In an embodiment depicted by the flow in Figure 11, (802) generating at at least one surface region of the insertable medical element an evanescent field having properties selected to substantially disable biomaterial in the at least one surface region may further include (1102) directing ultraviolet energy through a portion of the insertable medical element and generating the evanescent field responsive to the ultraviolet energy, (1104) wherein the ultraviolet energy includes UVC energy, and where the method may further comprise (1106) inhibiting transmission of the ultraviolet energy out of the insertable medical element, for example, with a reflector, an absorber, or a different device.

In one case, (1202) the evanescent field properties may include an energy range, where the method may further comprise (1204) varying the energy range, and (1206) wherein the energy range is selected such that a portion of the evanescent field is supported by an interface between air and the at least one surface region and/or (1208) wherein the energy range is selected such that a portion of the evanescent field is supported by an interface between the biomaterial and the at least one surface region.

In one case, (1302) the evanescent field properties include a spatial distribution, where the method may further comprise (1304) varying the spatial distribution, where (1304) varying the spatial distribution may include illuminating different areas with electromagnetic energy or other ways of changing the spatial distribution of the evanescent field. In another case, (1306) the evanescent field properties include an excitation duration, where the method may further comprise (1308) varying the excitation duration, where (1308) varying the excitation duration may include setting a pulse duration for electromagnetic energy incident on the insertable medical element. In another case, (1310) the evanescent field properties include an amplitude, where the method may further comprise (1312) varying the amplitude, where (1312) varying the amplitude of the evanescent field may include varying the amplitude of electromagnetic energy incident on the insertable medical element.

The method may further comprise (1402) determining an exposure for sterilization, (1404) determining an evanescent field amplitude and an evanescent field excitation duration for sterilization, (1406) determining an evanescent field energy range for sterilization and/or (1408) generating at at least one surface region of the insertable medical element an evanescent field with the determined evanescent field amplitude and for the determined evanescent field excitation duration.

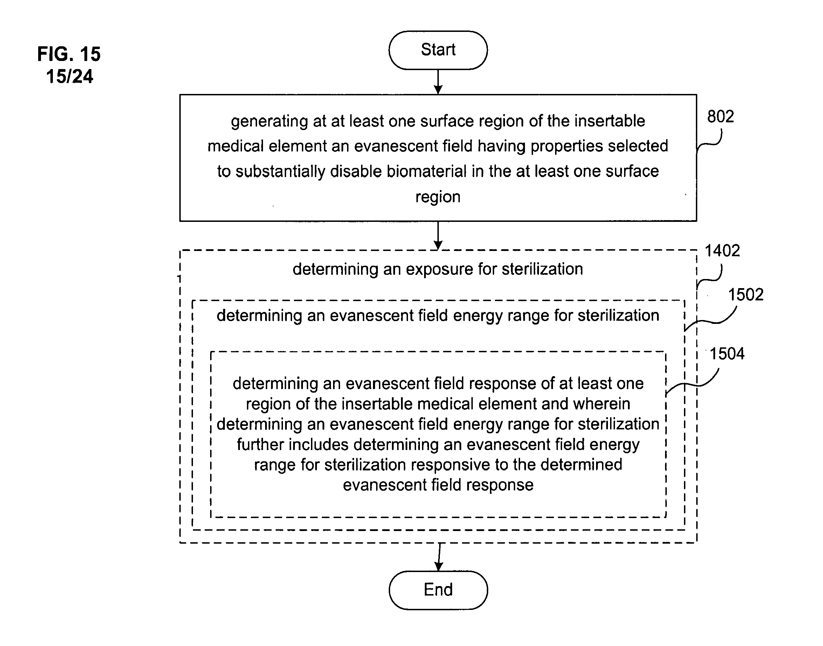

As shown in the flow of Figure 15, (1402) determining an exposure for sterilization may further comprise (1502) determining an evanescent field energy range for sterilization and (1504) determining an evanescent field response of at least one region of the insertable medical element and wherein determining an evanescent field energy range for sterilization further includes determining an evanescent field energy range for sterilization responsive to the determined evanescent field response.

In one case (1602) the insertable medical element includes a catheter. In another case, (1604) the insertable medical element includes a needle, where (1606) the insertable medical element may include a biopsy needle. In another case, (1608) the insertable medical element includes a shunt.

In one case, (1702) the at least one surface region of the insertable medical element includes an outer surface region of the insertable medical element. In another case, (1704) the at least one surface region of the insertable medical element includes a first inner surface region of the insertable medical element, (1706) wherein the at least one surface region of the insertable medical element may include a second inner surface region of the insertable medical element (where, for example, the insertable medical element includes a first and second biofluid guiding conduit 202, 302.)

The method may further comprise (1802) passing a first material through a first channel of the insertable medical element, (1804) wherein the first material may include the biomaterial. (1802) Passing the first material through a first channel of the insertable medical element may further include (1806) passing a second material through a second channel of the insertable medical element, and may further comprise (1810) passing the first material and the second material in substantially the same direction, (1812) passing the first material and the second material in substantially opposite directions, (1808) wherein the second channel is different from the first channel, and/or (1814) wherein the second material is different from the first material.

The method may further comprise (1902) receiving a signal indicative of the evanescent field properties (for example, the frequency and/or amplitude) and (1904) altering the evanescent field properties according to the received signal, (1906) wherein the signal indicative of the evanescent field properties may include information and the method may further comprise storing the information and (1908) transmitting the information (for example, electronically transmitting the information to a processor).

The method may further comprise (2002) generating at at least one surface region of the insertable medical element an evanescent field having properties selected to substantially disable biomaterial in response to user directives, (2004) sensing a property of the biomaterial (such as index of refraction), (2006) imaging the insertable medical element, (2008) generating the evanescent field in response to the presence of the biomaterial, and/or (2010) generating the evanescent field in response to a sensed parameter of the biomaterial. In one case, (2012) the biomaterial includes a pathogenic object. In another case, (2014) the biomaterial includes tissue.

In another embodiment, a method comprises (2102) guiding electromagnetic energy along a fluid passageway at least partially within a patient; (2104) generating plasmon energy along the fluid passageway responsive to the guided electromagnetic energy; and (2106) delivering a biomaterial through the fluid passageway after or during the generating plasmon energy along the fluid passageway responsive to the guided electromagnetic energy.

The method may further comprise (2202) passing a portion of the biomaterial through the plasmon energy, (2206) varying a first frequency range of the electromagnetic energy, (2208) generating plasmon energy outside the fluid passageway, and/or (2210) generating the plasmon energy via total internal reflection. In one case, (2204) the plasmon energy may substantially disable the biomaterial.

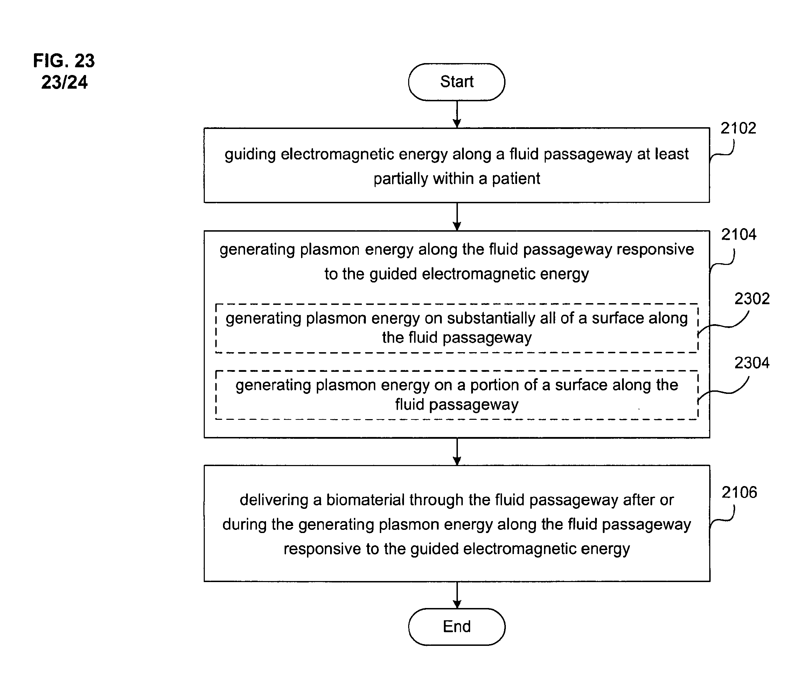

As shown in the flow of Figure 23, (2104) generating plasmon energy along the fluid passageway responsive to the guided electromagnetic energy may include (2302) generating plasmon energy on substantially all of a surface along the fluid passageway and/or (2304) generating plasmon energy on a portion of a surface along the fluid passageway.

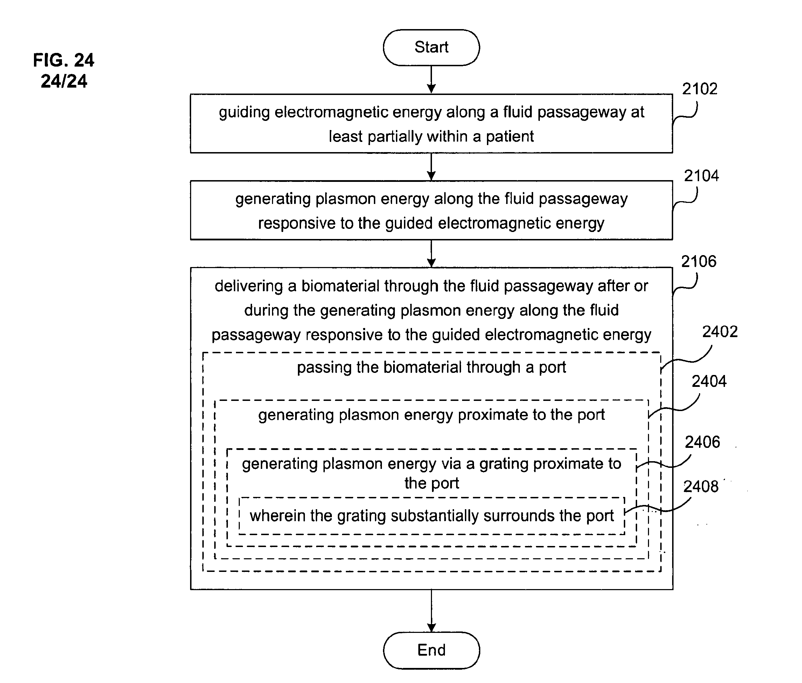

As shown in the flow of Figure 24, (2106) delivering a biomaterial through the fluid passageway after or during the generating plasmon energy along the fluid passageway responsive to the guided electromagnetic energy may include (2402) passing the biomaterial through a port (such as the aperture 602). The method may further comprise (2404) generating plasmon energy proximate to the port, where (2404) generating plasmon energy proximate to the port may further comprise (2406) generating plasmon energy via a grating proximate to the port, where in one case (2408) the grating substantially surrounds the port.

Those having skill in the art will recognize that the state of the art has progressed to the point where there is little distinction left between hardware and software implementations of aspects of systems; the use of hardware or software is generally (but not always, in that in certain contexts the choice between hardware and software can become significant) a design choice representing cost vs. efficiency tradeoffs. Those having skill in the art will appreciate that there are various vehicles by which processes and/or systems and/or other technologies described herein can be effected (e.g., hardware, software, and/or firmware), and that the preferred vehicle will vary with the context in which the processes and/or systems and/or other technologies are deployed. For example, if an implementer determines that speed and accuracy are paramount, the implementer may opt for a mainly hardware and/or firmware vehicle; alternatively, if flexibility is paramount, the implementer may opt for a mainly software implementation; or, yet again alternatively, the implementer may opt for some combination of hardware, software, and/or firmware. Hence, there are several possible vehicles by which the processes and/or devices and/or other technologies described herein may be effected, none of which is inherently superior to the other in that any vehicle to be utilized is a choice dependent upon the context in which the vehicle will be deployed and the specific concerns (e.g., speed, flexibility, or predictability) of the implementer, any of which may vary. Those skilled in the art will recognize that optical aspects of implementations will typically employ optically-oriented hardware, software, and or firmware.
The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or virtually any combination thereof. In one embodiment, several portions of the subject matter described herein may be implemented via Application Specific Integrated Circuits (ASICs), Field Programmable Gate Arrays (FPGAs), digital signal processors (DSPs), or other integrated formats. However, those skilled in the art will recognize that some aspects of the embodiments disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as a program product in a variety of forms, and that an illustrative embodiment of the subject matter described herein applies regardless of the particular type of signal bearing medium used to actually carry out the distribution. Examples of a signal bearing medium include, but are not limited to, the following: a recordable type medium such as a floppy disk, a hard disk drive, a Compact Disc (CD), a Digital Video Disk (DVD), a digital tape, a computer memory, etc.; and a transmission type medium such as a digital and/or an analog communication medium (e.g., a fiber optic cable, a waveguide, a wired communications link, a wireless communication link, etc.).

In a general sense, those skilled in the art will recognize that the various embodiments described herein can be implemented, individually and/or collectively, by various types of electro-mechanical systems having a wide range of electrical components such as hardware, software, firmware, or virtually any combination thereof; and a wide range of components that may impart mechanical force or motion such as rigid bodies, spring or torsional bodies, hydraulics, and electro-magnetically actuated devices, or virtually any combination thereof. Consequently, as used herein "electro-mechanical system" includes, but is not limited to, electrical circuitry operably coupled with a transducer (e.g., an actuator, a motor, a piezoelectric crystal, etc.), electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), electrical circuitry forming a communications device (e.g., a modem, communications switch, or optical-electrical equipment), and any non-electrical analog thereto, such as optical or other analogs. Those skilled in the art will also appreciate that examples of electro-mechanical systems include but are not limited to a variety of consumer electronics systems, as well as other systems such as motorized transport systems, factory automation systems, security systems, and communication/computing systems. Those skilled in the art will recognize that electro-mechanical as used herein is not necessarily limited to a system that has both electrical and mechanical actuation except as context may dictate otherwise.

In a general sense, those skilled in the art will recognize that the various aspects described herein which can be implemented, individually and/or collectively, by a wide range of hardware, software, firmware, or any combination thereof can be viewed as being composed of various types of "electrical circuitry." Consequently, as used herein "electrical circuitry" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

Those skilled in the art will recognize that it is common within the art to describe devices and/or processes in the fashion set forth herein, and thereafter use engineering practices to integrate such described devices and/or processes into image processing systems. That is, at least a portion of the devices and/or processes described herein can be integrated into an image processing system via a reasonable amount of experimentation. Those having skill in the art will recognize that a typical image processing system generally includes one or more of a system unit housing, a video display device, a memory such as volatile and non-volatile memory, processors such as microprocessors and digital signal processors, computational entities such as operating systems, drivers, and applications programs, one or more interaction devices, such as a touch pad or screen, control systems including feedback loops and control motors (e.g., feedback for sensing lens position and/or velocity; control motors for moving/distorting lenses to give desired focuses. A typical image processing system may be implemented utilizing any suitable commercially available components, such as those typically found in digital still systems and/or digital motion systems.

Those skilled in the art will recognize that it is common within the art to describe devices and/or processes in the fashion set forth herein, and thereafter use engineering practices to integrate such described devices and/or processes into data processing systems. That is, at least a portion of the devices and/or processes described herein can be integrated into a data processing system via a reasonable amount of experimentation. Those having skill in the art will recognize that a typical data processing system generally includes one or more of a system unit housing, a video display device, a memory such as volatile and non-volatile memory, processors such as microprocessors and digital signal processors, computational entities such as operating systems, drivers, graphical user interfaces, and applications programs, one or more interaction devices, such as a touch pad or screen, and/or control systems including feedback loops and control motors (e.g., feedback for sensing position and/or velocity; control motors for moving and/or adjusting components and/or quantities). A typical data processing system may be implemented utilizing any suitable commercially available components, such as those typically found in data computing/communication and/or network computing/communication systems.

All of the above U.S. patents, U.S. patent application publications, U.S. patent applications, foreign patents, foreign patent applications and non-patent publications referred to in this specification and/or listed in any Application Data Sheet, are incorporated herein by reference, to the extent not inconsistent herewith.

One skilled in the art will recognize that the herein described components (e.g., steps), devices, and objects and the discussion accompanying them are used as examples for the sake of conceptual clarity and that various configuration modifications are within the skill of those in the art. Consequently, as used herein, the specific exemplars set forth and the accompanying discussion are intended to be representative of their more general classes. In general, use of any specific exemplar herein is also intended to be representative of its class, and the non-inclusion of such specific components (e.g., steps), devices, and objects herein should not be taken as indicating that limitation is desired.

Although user 411 is shown/described herein as a single illustrated figure, those skilled in the art will appreciate that user 411 may be representative of a human user, a robotic user (e.g., computational entity), and/or substantially any combination thereof (e.g., a user may be assisted by one or more robotic agents). In addition, user 411, as set forth herein, although shown as a single entity may in fact be composed of two or more entities. Those skilled in the art will appreciate that, in general, the same may be said of "sender" and/or other entity-oriented terms as such terms are used herein.

With respect to the use of substantially any plural and/or singular terms herein, those having skill in the art can translate from the plural to the singular and/or from the singular to the plural as is appropriate to the context and/or application. The various singular/plural permutations are not expressly set forth herein for sake of clarity.

The herein described subject matter sometimes illustrates different components contained within, or connected with, different other components. It is to be understood that such depicted architectures are merely example, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected", or "operably coupled", to each other to achieve the desired functionality, and any two components capable of being so associated can also be viewed as being "operably couplable", to each other to achieve the desired functionality. Specific examples of operably couplable include but are not limited to physically mateable and/or physically interacting components and/or wirelessly interactable and/or wirelessly interacting components and/or logically interacting and/or logically interactable components.

While particular aspects of the present subject matter described herein have been shown and described, it will be apparent to those skilled in the art that, based upon the teachings herein, changes and modifications may be made without departing from the subject matter described herein and its broader aspects and, therefore, the appended claims are to encompass within their scope all such changes and modifications as are within the true spirit and scope of the subject matter described herein. Furthermore, it is to be understood that the invention is defined by the appended claims. It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean *at least* the recited number (e.g., the bare recitation of "two recitations," without other modifiers, typically means *at least* two recitations, or *two or more* recitations). Furthermore, in those instances where a convention analogous to "at least one of A, B, and C, etc." is used, in general such a construction is intended in the sense one having skill in the art would understand the convention (e.g., " a system having at least one ofA, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

With respect to the appended claims, those skilled in the art will appreciate that recited operations therein may generally be performed in any order. Examples of such alternate orderings may include overlapping, interleaved, interrupted, reordered, incremental, preparatory, supplemental, simultaneous, reverse, or other variant orderings, unless context dictates otherwise. With respect to context, even terms like "responsive to," "related to," or other past-tense adjectives are generally not intended to exclude such variants, unless context dictates otherwise.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

## Claims

1. An apparatus, comprising:
a first biofluid guiding conduit extending along a path from a first location to a second location, wherein at least a portion of the first biofluid guiding conduit is insertable into a patient;
a guiding structure configured to guide electromagnetic energy proximate to at least a portion of the path; and
a conversion structure operative to convert electromagnetic energy to plasmon energy, the conversion structure being positioned to receive the guided electromagnetic energy and to provide the plasmon energy to the portion of the path.

2. The apparatus of claim 1 wherein the guiding structure includes an optically transmissive material.

3. The apparatus of claim 2 wherein the optically transmissive material includes ultraviolet transmissive material.

4. The apparatus of any of claims 1 to 3 wherein the guiding structure includes an optical waveguide.

5. The apparatus of any preceding claim wherein the conversion structure includes a conductive coating.

6. The apparatus of claim 5 wherein the conductive coating includes silver.

7. The apparatus of any preceding claim further comprising a biocompatible material in intimate contact with a portion of the first biofluid guiding conduit.

8. The apparatus of any preceding claim wherein the first biofluid guiding conduit includes an inner wall surface and wherein the conversion structure covers at least a portion of the inner wall surface.

9. The apparatus of any preceding claim wherein the first biofluid guiding conduit includes at least one wall defining a passageway.

10. The apparatus of claim 9 wherein the at least one wall defining a passageway is configured to support electromagnetic energy transmission.

11. The apparatus of claim 10 wherein the at least one wall defining a passageway includes an ultraviolet transmission material.

12. The apparatus of any preceding claim further including:
an electromagnetic shield positioned to substantially block emission of the electromagnetic energy external to the apparatus.

13. The apparatus of any preceding claim further comprising a second fluid guiding conduit arranged proximate to the first biofluid guiding conduit.

14. The apparatus of any preceding claim wherein the guiding structure substantially surrounds the first biofluid guiding conduit.

15. The apparatus of any preceding claim wherein the guiding structure is substantially parallel to the first biofluid guiding conduit.

16. The apparatus of any preceding claim further comprising a sensor.

17. The apparatus of any preceding claim further comprising a generator arranged to produce the electromagnetic energy.

18. The apparatus of any preceding claim further comprising a receiver positioned to receive energy.

19. The apparatus of claim 18 wherein the energy includes the guided electromagnetic energy.

20. The apparatus of claim 18 or claim 19 wherein the energy includes the plasmon energy.

21. The apparatus of any of claims 18 to 20 further comprising a transmitter arranged to transmit information related to the received energy.

22. The apparatus of any of claim 18 to 21 further comprising a storage medium arranged to store information related to the received energy.

23. The apparatus of any of claims 18 to 22 further comprising a processor arranged to process information related to the received energy.

24. The apparatus of any of claims 18 to 24 further comprising an input port arranged to receive a first signal directive of information related to the received energy.

25. The apparatus of claim 24 wherein the first signal includes a user selection.

26. The apparatus of any preceding claim wherein the conversion structure includes a grating.

27. The apparatus of any preceding claim wherein the conversion structure includes a total internal reflection interface.

28. A system comprising:
a light source having an output energy;
an insertable medical element supportive of evanescent energy; and
an evanescent field generator coupled to receive the output energy and responsive to produce the evanescent energy within or proximate a biomaterial at a sterilization level.

29. The system of claim 28 further comprising a waveguide.

30. The system of claim 29 wherein the waveguide includes an optical fiber.

31. The system of claim 30 wherein the insertable medical element includes the waveguide.

32. The system of any of claims 28 to 31 wherein the evanescent energy includes plasmon energy.

33. The system of any of claims 28 to 32 wherein the evanescent energy is plasmon energy.

34. The system of any of claims 28 to 33 further comprising a fluid guide.

35. The system of any of claims 34 wherein the fluid guide is arranged to carry the biomaterial.

36. The system of claim 34 or claim 35 wherein the fluid guide includes an aperture.

37. The system of claim 36 further comprising a grating proximate to the aperture.

38. The system of claim 36 or claim 37 wherein the evanescent field generator is proximate to the aperture.

39. The system of any of claims 28 to 38 wherein the evanescent energy is selected to substantially disable the biomaterial.

40. The system of any of claims 28 to 39 wherein the evanescent field generator includes a grating.

41. The system of any of claim 28 to 40 wherein the insertable medical element includes a catheter.

42. The system of any of claims 28 to 41 wherein the insertable medical element includes a needle.

43. The system of claim 42 wherein the needle includes a biopsy needle.

44. The system of any of claims 28 to 43 wherein the insertable medical element includes an implantable device.

45. The system of any of claims 28 to 44 wherein the insertable medical element includes a shunt.

46. The system of any of claims 28 to 45 further comprising a sensor.

47. The system of claim 46 wherein the sensor includes a surface plasmon resonance sensor.

48. The system of any of claims 28 to 47 further comprising an imaging system.

49. The system of claim 48 wherein the imaging system is operable to image the insertable medical element.

50. The system of claim 48 or claim 49 wherein the imaging system includes an optical imaging system.

51. The system of any of claim 48 to 50 wherein the imaging system includes an ultrasound system.

52. The system of any of claims 48 to 51 wherein the imaging system includes an MRI system.

53. The system of any of claim 48 to 52 wherein the imaging system includes a radiography system.

54. The system of any of claims 48 to 53 wherein the imaging system is operative to detect biomaterial.

55. The system of any of claims 48 to 55 wherein the insertable medical element includes a portion of the imaging system.

56. A method of establishing a sterile region proximate to an insertable element, comprising:
generating at at least one surface region of the insertable element an evanescent field having properties selected to substantially disable biomaterial in the at least one surface region.

57. The method of claim 58 further including:
shielding a region external to the insertable element from electromagnetic energy.

58. The method of claim 58 or claim 59 further comprising blocking the evanescent field at at least one location on the at least one surface region of the insertable element.

59. The method of any of claims 56 to 58 wherein the evanescent field includes a plasmon field.

60. The method of any of claims 56 to 59 wherein the evanescent field is a plasmon field.

61. The method of any of claim 56 to 60 wherein generating at at least one surface region of the insertable element an evanescent field includes:
directing ultraviolet energy through a portion of the insertable element; and
generating the evanescent field responsive to the ultraviolet energy.

62. The method of claim 61 wherein the ultraviolet energy includes UVC energy.

63. The method of claim 61 or claim 62 further including inhibiting transmission of the ultraviolet energy out of the insertable element.

64. The method of any of claims 56 to 63 wherein the evanescent field properties include an energy range.

65. The method of claim 64 further comprising varying the energy range.

66. The method of claim 64 or claim 65 wherein the energy range is selected such that a portion of the evanescent field is supported by an interface between air and the at least one surface region.

67. The method of any of claims 64 to 66 wherein the energy range is selected such that a portion of the evanescent field is supported by an interface between the biomaterial and the at least one surface region.

68. The method of any of claims 56 to 67 wherein the evanescent field properties include a spatial distribution.

69. The method of claim 68 further comprising varying the spatial distribution.

70. The method of any of claims 56 to 69 wherein the evanescent field properties include an excitation duration.

71. The method of claim 70 further comprising varying the excitation duration.

72. The method of any of claim 56 to 71 wherein the evanescent field properties include an amplitude.

73. The method of claim 72 further comprising varying the amplitude.

74. The method of any of claims 56 to 73 further comprising determining an exposure for sterilization.

75. The method of claim 74 wherein determining an exposure for sterilization includes determining an evanescent field amplitude and an evanescent field excitation duration for sterilization.

76. The method of claim 75 wherein determining an exposure for sterilization further includes determining an evanescent field energy range for sterilization.

77. The method of claim 75 or claim 76 wherein generating at at least one surface region of the insertable element an evanescent field includes generating at at least one surface region of the insertable element an evanescent field with the determined evanescent field amplitude and for the determined evanescent field excitation duration.

78. The method of any of claims 74 to 77 wherein determining an exposure for sterilization includes determining an evanescent field energy range for sterilization.

79. The method of claim 78 further comprising determining an evanescent field response of at least one region of the insertable element and wherein determining an evanescent field energy range for sterilization further includes determining an evanescent field energy range for sterilization responsive to the determined evanescent field response.

80. The method of any of claims 56 to 79 wherein the at least one surface region of the insertable element includes an outer surface region of the insertable element.

81. The method of any of claims 56 to 80 wherein the at least one surface region of the insertable medical element includes a first inner surface region of the insertable medical element.

82. The method of any of claims 56 to 81 wherein the at least one surface region of the insertable medical element includes a second inner surface region of the insertable medical element.

83. The method of any of claims 56 to 82 further comprising passing a first material through a first channel of the insertable element.

84. The method of claim 83 wherein the first material includes the biomaterial.

85. The method of claim 83 or claim 84 further comprising passing a second material through a second channel of the insertable element.

86. The method of claim 85 wherein the second channel is different from the first channel.

87. The method of claim 85 or claim 86 further comprising passing the first material and the second material in substantially the same direction.

88. The method of claim 85 or claim 86 further comprising passing the first material and the second material in substantially opposite directions.

89. The method of any of claims 85 to 88 wherein the second material is different from the first material.

90. The method of any of claims 56 to 89 further comprising receiving a signal indicative of the evanescent field properties.

91. The method of claim 90 further comprising altering the evanescent field properties according to the received signal.

92. The method of claim 90 or claim 91 wherein the signal indicative of the evanescent field properties includes information and further comprising storing the information.

93. The method of claim 92 further comprising transmitting the information.

94. The method of any of claims 56 to 93 further comprising generating at at least one surface region of the insertable element an evanescent field having properties selected to substantially disable biomaterial in response to user directives.

95. The method of any of claims 56 to 94 further comprising sensing a property of the biomaterial.

96. The method of any of claim 56 to 95 further comprising imaging the insertable element.

97. The method of any of claims 56 to 96 further comprising generating the evanescent field in response to the presence of the biomaterial.

98. The method of any of claims 56 to 98 further comprising generating the evanescent field in response to a sensed parameter of the biomaterial.

99. The method of any of claims 56 to 98 wherein the biomaterial includes a pathogenic object.

100. The method of any of claim 56 to 99 wherein the biomaterial includes tissue.

101. A method, comprising:
guiding electromagnetic energy along a fluid passageway;
generating plasmon energy along the fluid passageway responsive to the guided electromagnetic energy; and
delivering a biomaterial through the fluid passageway after or during the generating plasmon energy along the fluid passageway responsive to the guided electromagnetic energy.

102. The method of claim 101 further comprising passing a portion of the biomaterial through the plasmon energy.

103. The method of claim 101 or claim 102 wherein the plasmon energy substantially disables the biomaterial.

104. The method of any of claims 101 to 103 wherein the fluid passageway includes a surface, and wherein generating plasmon energy along the fluid passageway includes generating plasmon energy on substantially all of the surface along the fluid passageway.

105. The method of any of claims 101 to 104 wherein the fluid passageway includes a surface, and wherein generating plasmon energy along the fluid passageway includes generating plasmon energy on a portion of the surface along the fluid passageway.

106. The method of any of claims 101 to 105 wherein the electromagnetic energy has a first frequency range and further comprising varying the first frequency range.

107. The method of any of claims 101 to 106 further comprising generating plasmon energy outside the fluid passageway.

108. The method of any of claims 101 to 107 further comprising generating the plasmon energy via total internal reflection.

109. The method of any of claims 101 to 108 wherein delivering a biomaterial to a patient through the fluid passageway includes passing the biomaterial through a port.

110. The method of claim 109 further comprising generating plasmon energy proximate to the port.

111. The method of claim 109 or claim 110 wherein generating plasmon energy proximate to the port includes generating plasmon energy via a grating proximate to the port.

112. The method of claim 111 wherein the grating substantially surrounds the port.
